# EUROPEAN PATENT APPLICATION

(11) **EP 1 688 428 A1**
(43) Date of publication of application: **09.08.2006**
(21) Application number: 04797391.2
(22) Date of filing: 03.11.2004
(51) Int. Cl.: C07K 14/22, A61K 39/00

(54) **METHOD OF ANTIGEN INCORPORATION INTO NEISSERIA BACTERIAL OUTER MEMBRANE VESICLES AND RESULTING VACCINE FORMULATIONS**

(30) Priority: 04.11.2003 CU 25403
(71) Applicant: CENTRO DE INGENIERIA GENETICA Y BIOTECNOLOGIA, Ciudad de La Habana 10600 (CU)
(72) Inventor: NIEBLA PEREZ, Olivia, Calle: 186, La Habana 12100 (CU); PAJON FEYT, Rolando, La Habana 32400 (CU); GONZALEZ BLANCO, Sonia, Calle 186, La Habana 12100 (CU); MARTIN DUNN, Alejandro, Miguel, Calle 31, La Habana 12100 (CU); DELGADO ESPINA, Maité, Calle 186, La Habana 12100 (CU); GARAY PEREZ, Hilda, Elisa, Avenida 31, La Habana 12100 (CU); GUILLEN NIETO, Gerardo, Enrique, Calle Linea 6, La Habana 10400 (CU)
(74) Representative: van Loon, C.J.J.
(86) International application number: PCT/CU2004/000012
(87) International publication number: WO 2005/042571

(57) **Abstract**

Method for the insertion of protein antigens, of recombinant or synthetic origin, in outer membrane vesicles of Gram-negative bacteria without disruption of the vesicle structure, therefore maintaining the immunogenicity and immunostimulatory properties of said vesicles, and with the reported advantage that the immune response generated against the incorporated antigen is superior to the one generated when the antigen is administered alone. The resultant vaccine formulations are useful to increase protective capacity of existing vaccines and allow to extend it against different pathogens, in diseases of bacterial, viral, cancerous or other etiology.

The referred formulations are applicable in the pharmaceutical industry as vaccines for therapeutic and preventive use in humans.

## Description

### Field of the Invention

The present invention is related to the field of medicine, particularly with the development of vaccine formulations for preventive or therapeutic use, that allow the increase in the quality of the immune response generated against vaccine antigens in diseases of different origins.

### Background of the Invention (Previous State of the art)

Recombinant DNA technology has brought an enormous advance in the field of vaccine research by making possible to obtain substantial amounts of several antigens and vaccine candidates which in turn speed up the testing of its immunogenicity and their potential as protective character. However, most of the time, this proteins are produced as inclusion bodies.

Bacterial inclusion bodies are protein aggregates of unfolded proteins, that are produced by transformed bacteria after the over expression of the cloned genes. In biotechnology the formation of these inclusion bodies, even when they allow a high recovery and production of the recombinant protein, pose a threat on the final goal that is to obtain a properly folded, biologically active, protein product. The recovery process from these aggregates usually involves complex steps of refolding (Carrio M.M., Villaverde A. (2002) Construction and deconstruction of bacterial inclusion bodies. *J Biotechnol.* 96:3-12).

### Protein folding

Within the last decade several strategies in order to deal with extraction and re-folding of proteins produced as inclusion bodies have been devised. As a normal trend these inclusion bodies can be easily separated and solubilized with chaotropic agents such as guanidinium chloride, or urea, and subsequently refolded by dialysis. Recent advances in refolding techniques include the application of low molecular weight additives and matrix-based folding (Misawa S. and Kumagai I. (1999) Refolding of therapeutic proteins produced in *Escherichia coli* as inclusion bodies. *Biopolymers* 51:297-307).

In order to purify and refold an outer membrane mitocondrial porin, which forms an anionic gated channel, a single step using metal chelating chromatography was employed. In this procedure the soluble fraction upon protein solubilization is applied onto the column and corresponding contaminants are washed with n-octyl -beta-D-glucopyranoside (OG) and glycerol. The fraction containing the protein of interest is then eluted in the presence of OG and imidazole (Shi Y., et al. (2003) One-step on-column affinity refolding purification and functional analysis of recombinant human VDAC1. *Biochem Biophys Res Común.* 303:475-82).

In a second example the P2 protein from *Haemophilus influenzae,* one of the most immunogenic, and a major protein in the bacterial outer membrane, was folded in a solution with high ionic strength and calcium (Pullen J.K., et al. (1995). Production of *Haemophilus influenzae* type-b porin in *Escherichia coli* and its folding into the trimeric form. Gene 152:85-8).

Porin protein from *Rhodobacter capsulatus,* non-recombinant, was chemically modified with methoxypoly(ethylene glycol) succinimidyl carbonate, rendering a soluble conjugate. The refolding of this conjugate was analyzed by the sequential addition of trifluoroethanol in order to unsuccessfully get a low dielectric constant. Finally, the protein was refolded after the addition of 5 to 10% of hexafluoro-2-propanol (Wei J., Fasman G.D. (1995) A poly(ethylene glycol) water-soluble conjugate of porin: refolding to the native state. *Biochemistry* 34:6408-15).

Two different types of PorA proteins from meningococci, P1.6 and P1.7,16 were folded *in vitro* after over-expression and purification from *Escherichia coli.* These proteins were refolded by fast dilution into a buffered solution containing n-dodecyl-N, N-dimethyl-1-ammonio-3-propanesulphonate (Jansen C., et al. (2000) Biochemical and biophysical characterization of in vitro folded outer membrane porin PorA of *Neisseria meningitidis. Biochim Biophys* 1464:284-98).

The insertion into lipid membranes is a widely used strategy to fold porins and other integral membrane proteins generated by genetic engineering. The major protein of *Chlamydia psittaci* and *Chlamydia pneumoniae* outer membranes were solubilized from inclusion bodies using 2% OG and 1 mM dithiotreitol, before being incorporated into a lipid bilayer (Wyllie S., et al. (1999) Single channel analysis of recombinant major outer membrane protein porins from *Chlamydia psittaci* and *Chlamydia pneumoniae. FEBS Lett* 445:192-96).

Outer membrane proteins from *E. coli,* OmpF and OmpA were re-folded by dilution in a colloidal solution of lipid vesicles and/or detergent/lipid vesicles (Surrey T., et al. (1996). Folding and membrane insertion of the trimeric beta-barrel protein OmpF. *Biochemistry* 35: 2283-88).

The *Neisserial opc* gene was cloned and expressed at high levels in *E. coli.* The protein was purified by affinity chromatography and was subsequently incorporated into liposomes and detergent micelles. in order to increase the immune response against the recombinant protein, Mono-phosphoryl Lipid A (MPLA) was added to the liposomes increasing the magnitude and quality of the elicited immune response. The functionality of the response was higher in the group where liposomes and

MPLA were used (Kolley K.A., et al. (2001) Immunization with Recombinant Opc Outer Membrane Protein from *Neisseria meningitidis:* Influence of Sequence Variation and Levels of Expression on the Bactericidal Immune Response against Meningococci. *Infect Immun.* 69:3809-16).

PorA and PorB proteins from *N. meningitidis,* obtained from recombinant hosts, have been re-folded using the same strategy of incorporation onto liposomes of phopholipids and cholesterol with the eventual use of detergents.

The *porA* gene from *N. meningitidis* was cloned and expressed in *E. coli.* The purified protein was used as immunogen in the presence of AI(OH)₃, or different adjuvants as liposomes. The immunization induced high avidity antibodies against the native protein that were able to react with live meningococci and inhibited the action of protective antibodies (Christodoulides M., et al. (1998) Immunization with recombinant class 1 outer-membrane protein from *Neisseria meningitidis:* influence of liposomes and adjuvants on antibody avidity, recognition of native protein and the induction of a bactericidal immune response against meningococci. *Microbiology* 144:3027-37).

The *porB* gene from a *N. meningitidis* strain expressing the PorB3 protein serotype was cloned and inserted into the pRSETB cloning vector and the correspondent protein was expressed at high levels in the *E. coli* host. The recombinant protein was purified by affinity chromatography and was used for animal immunization after its incorporation into liposomes and detergent micelles (Zwittergent or sulfobetaine). The sera elicited by liposomes and micelles showed the highest reactivity against the native protein (Wright J.C., et al (2002). Immunization with the Recombinant PorB Outer Membrane Protein Induces a Bactericidal Immune Response against *Neisseria meningitidis. Infect. Immun.* 70:4028-34).

### Neisseria meningitidis, vesicles and outer membrane proteins

*Neisseria meningitidis,* is a Gram-negative diplococcus whose only natural host is man, is a well known causal agent of bacterial meningitis. Normally, this bacterium is carried by asymptomatic carriers in the population, being this way the most common for coiiection. So far, several strategies have been developed in order to obtain a vaccine capable of protecting people of this devastating disease. In this sense, capsular polysaccharide, that usually allows the classification of this bacterium into serogroups, has been employed. However, the serogroup B is still a significant source of endemic, as well as epidemic, meningococcal disease due to the lack of an effective vaccine against it. As a result of its low immunogenicity (Finne J., et al. (1987) An IgG monoclonal antibody to group B meningococci cross-reacts with developmentally regulated polysialic acid units of glycoproteins in neural and extra neural tissues. *J. Immunol.* 138:4402-07), the development of vaccine against this serogroup is focused in sub capsular antigens.

During the 70's the production of outer membrane protein (OMP) based vaccines was based in the elimination of the lipopolysaccharide (LPS) from the protein preparation by means of sequential detergent extrations (Frasch C.E., Robbins J.D. (1978) Immunogenicity of serotype 2 vaccines and specificity of protection in a guinea pig model. *J. Exp. Med.* 147(3):629-44), and followed by salting out precipitation of the OMPs. Despite the good results obtained in animals, these vaccines failed to induce bactericidal antibodies in young adults and children (Zollinger W.D., et al. (1978) Safety and immunogenicity of a *Neisseria meningitidis* type 2 protein vaccine in animals and humans. *J. Infect. Dis.* 137(6):728-39), a result that was explained due to protein denaturation in the preparation after the precipitation step. Subsequent attempts were directed to design a vaccine with proteins in their native folded state, by using outer membrane vesicles (OMV) (Zollinger W.D., et al. (1979) Complex of meningococcal group B polysaccharide and type 2 outer membrane protein immunogenic in man. *J*. *Clin. Invest.* 63(5):836-48; Wang L.Y. and Frasch C.E. (1984) Development of a *Neisseria meningitidis* group B serotype 2b protein vaccine and evaluation in a mouse model. *Infect Immun.* 46(2):408-14).

OMV based vaccines were significantly more immunogenic by parenteral route than the aggregated OMPs and the initial success was attributed to a higher adsorption onto Al(OH)₃ adjuvant (Wang L.Y. and Frasch C.E. (1984) Development of a *Neisseria meningitidis* group B serotype 2b protein vaccine and evaluation in a mouse model. *Infect Immun.* 46(2):408-14). However, it eventually became apparent that the effectiveness showed is attributable, by a large extent, to the presentation of the OMPs in their native conformation, allowing the induction of a potent bactericidal immune response in teenagers and adults. The generated antibody responses increased the opsonophagocytosis of meningococci. The exact formulation of these vaccines (i.e. OMP content, LPS content, and adjuvant) has a significant impact on its immunogenicity, although there are big differences between the producers according to the strain and/or the employed methodology (Lehmann A.K., et al. (1991) Immunization against serogroup B meningococci. Opsonin response in vaccinees as measured by chemiluminescence. *APMIS* 99(8):769-72; Gomez J.A., et al. (1998) Effect of adjuvants in the isotypes and bactericidal activity of antibodies against the transferrin-binding proteins of *Neisseria meningitidis. Vaccine* 16(17):1633-39; Steeghs L., et al. (1999) Immunogenicity of outer membrane proteins in a lipopolysaccharide-deficient mutant of *Neisseria meningitidis:* influence of adjuvants on the immune response. *Infect. Immun.* 67(10):4988-93).

Among the most studied vaccine candidates against meningococcus we have the major porins. Of them, PorA protein, of 42 kDa aproximately and by far the most important, have shown to exhibit a high degree of sequence variability, mainly in two of the 8 exposed loops (VR1, VR2). Variability in this regions is the cause of the present strain subtyping method of neisserial strains (Abdillahi H. and Poolman J.T. (1988) *Neisseria meningitidis* group B serosubtyping using monoclonal antibodies in whole-cell ELISA. *Microb. Pathog.* 4:27-32). With the use of synthetic peptides and monoclonal antibodies, immunodominant epitopes have been mapped to be located on these very same regions (McGuinness B., Lambden P.R. and Heckels J.E. (1993) Class 1 outer membrane protein of *Neisseria meningitidis:* epitope analysis of the antigenic diversity between strains, implications for subtype definition and molecular epidemiology. *Mol. Microbiol.* 7:505-514).

Although these epitopes are linear, when these proteins are cloned and expressed in *E. coli* (Niebla O. (2001) Immunogenicity of recombinant class 1 protein from *Neisseria meningitidis* refolded into phospholipid vesicles and detergent. *Vaccine* 19:3568-74) or any other host like *Bacillus subtilis* (Nurminen M., et al. (1992) The class 1 outer membrane protein of *Neisseria meningitidis* produced in *Bacillus subtilis* can give rise to protective immunity. *Mol. Microbiol.* 6:2499-2506), the refolding process plays a critical role on the immune response generation, since the induction of bactericidal and protective antibodies greatly relies on the satisfactory presentation to the immune system. This fact explains the existent demand of proper re-folding for some antigens which constitutes a state of the art problem for which we are actively looking for solutions.

### Description of the invention

The present invention solves the problem previously mentioned by offering a method for the incorporation of antigens into OMVs, where these antigens form, by co-folding, a complex with this preparation of outer membrane proteins of Gram-negative bacteria, while maintaining intact the vesicle structure of the OMV.

In the preferred realization the method includes a preparation of outer membrane proteins from Gram-negative bacteria, obtained from species belonging to *Neisseriaceae* family, or Bramhamella catarrhalis being the specially preferred those including *N. meningitidis* and *N*. *lactamica* OMVs.

In an additional materialization of the invention the protein antigen is of natural origin, as well as recombinant or synthetic.

Equally the invention refers to the vaccine combination derived from the method described here and it encompass a complex formed by the preparation of Gram-negative outer membrane protein preparations, generated from species belonging to the *Neisseriaceae* family or to *Bramhamella catarrhalis,* and a protein antigen of natural, recombinant, or synthetic origin, where this complex is generated by co-folding while maintaining intact the vesicle structure of the OMV. Depending on the circumstances, these vaccine formulations can be administered by parenteral or mucosal routes.

A particularly important aspect of the said invention is related to the addition of bacterial polysaccharides, conjugated or not, and nucleic acids, as antigens to the previously mentioned vaccine preparations.

It is also part of the present invention the prophylactic or therapeutic use of the described combinations in humans.

In these compositions, the protein antigens are folded by their insertion into the OMV, a process that allows the proper folding of said antigens. These formulations have new inherent properties related to the generation of the complex, formed in such a way that the vesicle structure of the OMVs remains intact.

Mucosal administration of such compositions is able to induce a systemic immune response of similar intensity and higher quality to the one generated with conventional vaccine formulations using Al(OH)₃ as an adjuvant. Additionally, the immunization through this route is able to elicit a potent mucosal immune response characterized by high levels of antibodies of the IgA subclass.

The incorporation of recombinant PorA protein into meningococcal OMVs increases the protective spectrum of the said OMVs, while facilitating the re-folding of this antigen, and allowing the induction of subtype specific antibodies capable to promote the complement-mediated lysis of the bacteria.

The present invention, in contrast with the previous state of the art, is useful to achieve the proper folding of protein antigens that would, otherwise, require their inclusion into artificial lipid bilayers, chemical modification, or mix with inorganic additives. Through this invention the immune response generated is better, in terms of quality of the antibodies elicited against the target antigen, due to the optimal presentation to the immune system.

### Brief description of drawings

In part, the main results are showed graphically in the following figures:

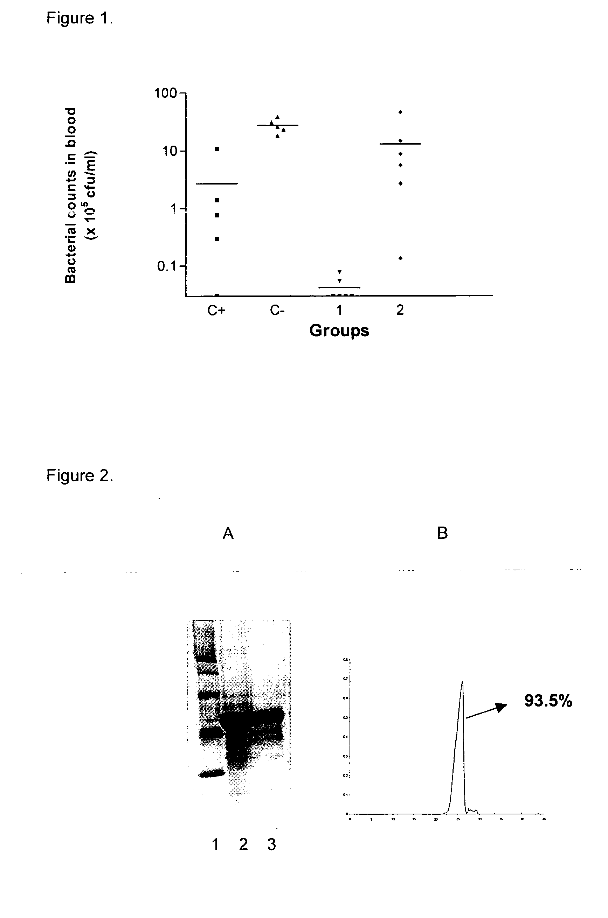

Figure 1: Passive protection against meningococcal infection, determined in the infant rat model of bacteremia. Pups received pooled sera from mice immunized with: 1. OMVs 2. Denatured OMVs (no vesicles). As negative control (C-), a serum from a non-immune mouse was used, and as positive control (C+) hyperimmune sera from mice immunized by parenteral route with OMVs.
Figure 2: Purification of recombinant PorA. **A**: 10% SDS PAGE. Lane 1: molecular weight marker. Lane 2: Starting sample, fraction collected after sonication. Lane 3: Final sample, eluted from ionic exchange. **B**: Chromatogram of the last ionic exchange purification.

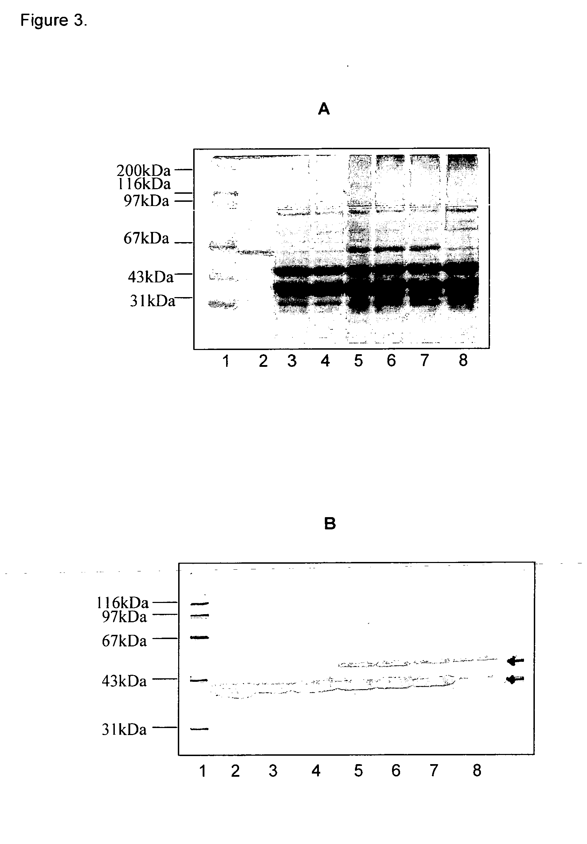

Figure 3: Electrophoretic analysis of different incorporation strategies. **A.** 10% SDS-PAGE. Lane 1: molecular weight marker. Lane 2: Recombinant Protein. Lanes 3,4,5,6,7: incorporation variants. Lane 8: OMVs. **B**: Immunoidentification of the incorporated protein with monoclonal antibody anti-P1.16, by Western blot. Lane 1: molecular weight marker. Lane 2: VME. Lanes 3,4,5,6,7: incorporation variants. Lane 8: recombinant protein.

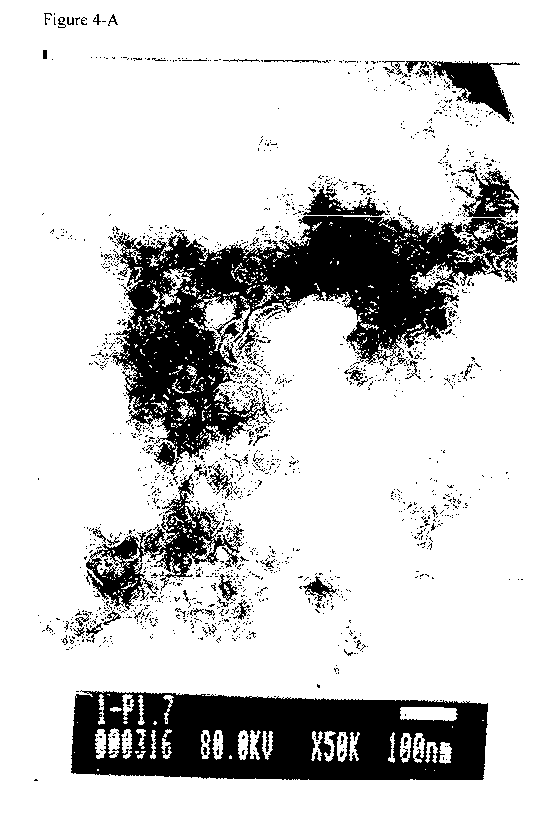

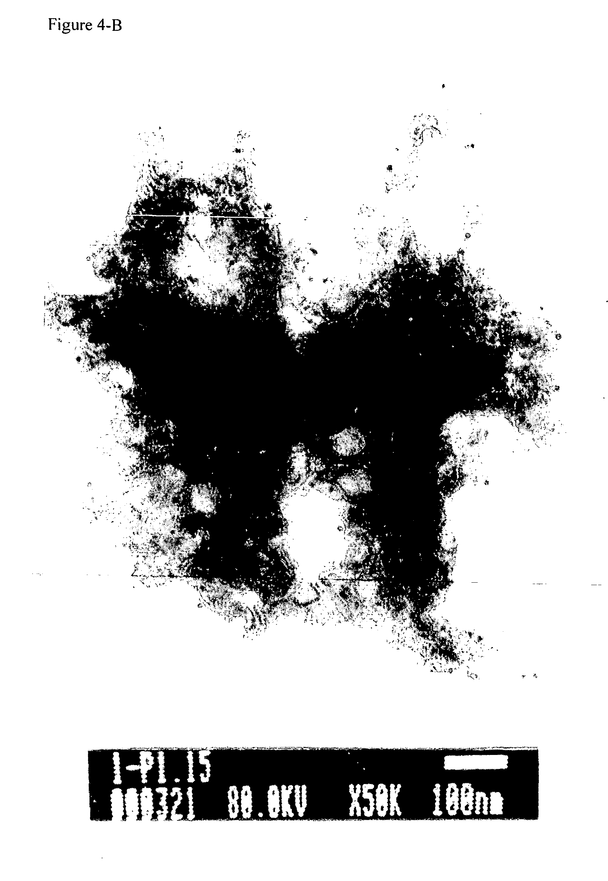

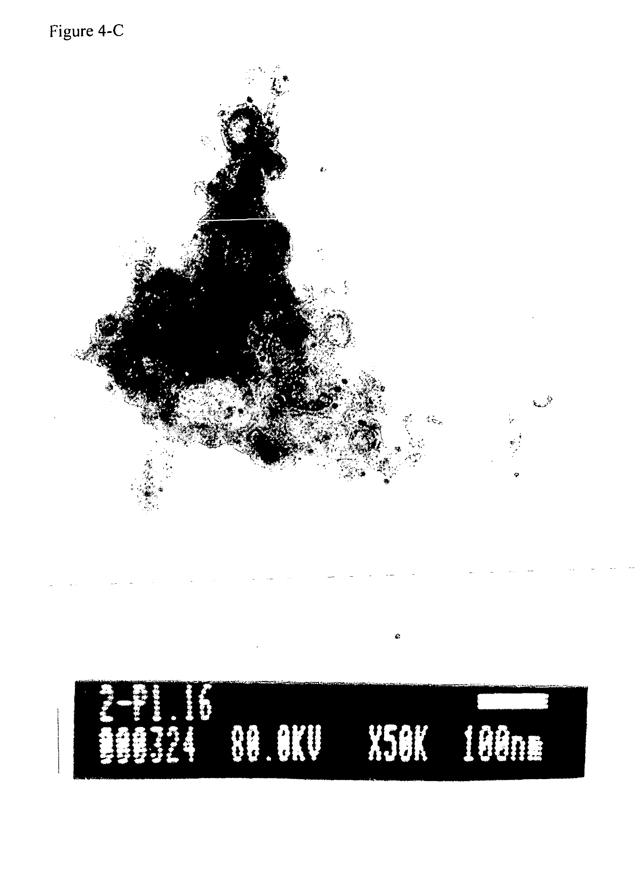

Figure 4. Electron microscopy visualizations showing the insertion of the recombinant protein PorA 7,16,9. **A:** Outer membrane vesicles treated by negative staining and transmission electron microscopy, strain CU385. **B:** OMVs tagged through gold-labeled MAb anti-P1.15. **C:** OMVs tagged through gold-labeled MAb anti-P1.16.

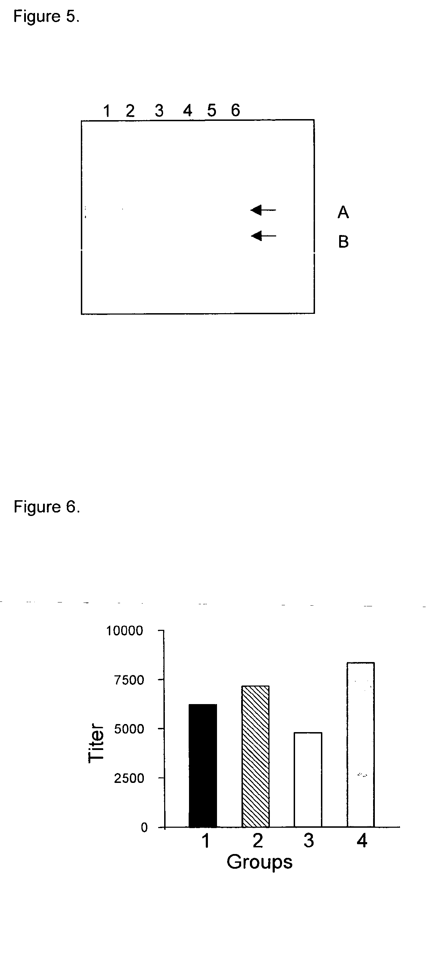

Figure 5. Western blot performed to test protein incorporation under different conditions. **A:** recombinant P1. 7,16,9 detected with Mab 1-33, anti-P1.9. **B:** Natural P1.15 present in the vesicle. Lane 1: Non modified OMV. Lane 2: Purified recombinant protein. Lane 3,4,5,6: incorporation variants.
Figure 6. Graphic representation of the IgG titers against recombinant protein P1.7,16,9, raised in the different groups of animals immunized with: **1**: recombinant P1.9, **2**: recombinant P1.16, **3**: recombinant P1.7,16, **4**: recombinant P1.7,16,9. The titer was calculated as the reciprocal value of the maximal dilution that triplicates the Optical Density (O.D) of the pre-immune sera.

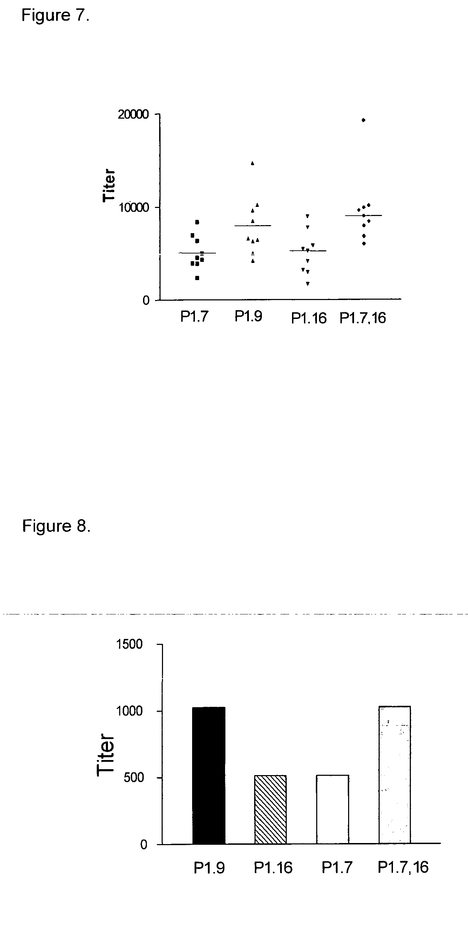

Figure 7. Graphic representation of individual animal titers (IgG) against neisserial porins, with recombinant P1.7,16,9 used as an immunogen. The titer was calculated as the reciprocal value of the maximal dilution that triplicates the O.D of the pre-immune sera.
Figure 8. Graphic representation of bactericidal titers of the sera raised by immunization with recombinant P1. 7,16,9, against strains bearing these very same subtypes. The final titer was calculated as the reciprocal value of the maximal dilution that produces 50% killing of the total bacteria applied.

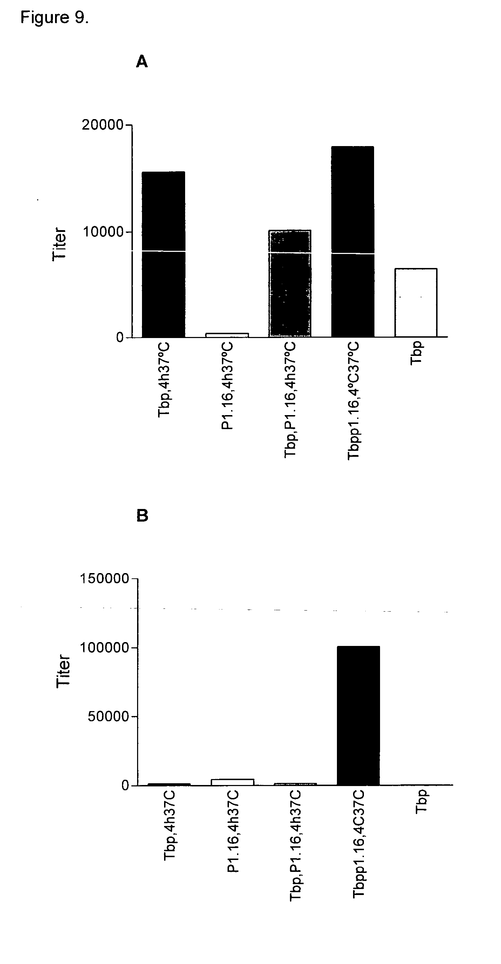

Figure 9. Graphical representation of titers against recombinant TbpB protein generated in the different variants assayed. A- Evaluation against the recombinant protein. B- Evaluation against the natural protein contained in the OMVs of strain B16B6. The titer was calculated as the reciprocal value of the maximal dilution that duplicates the O.D of the pre-immune sera.

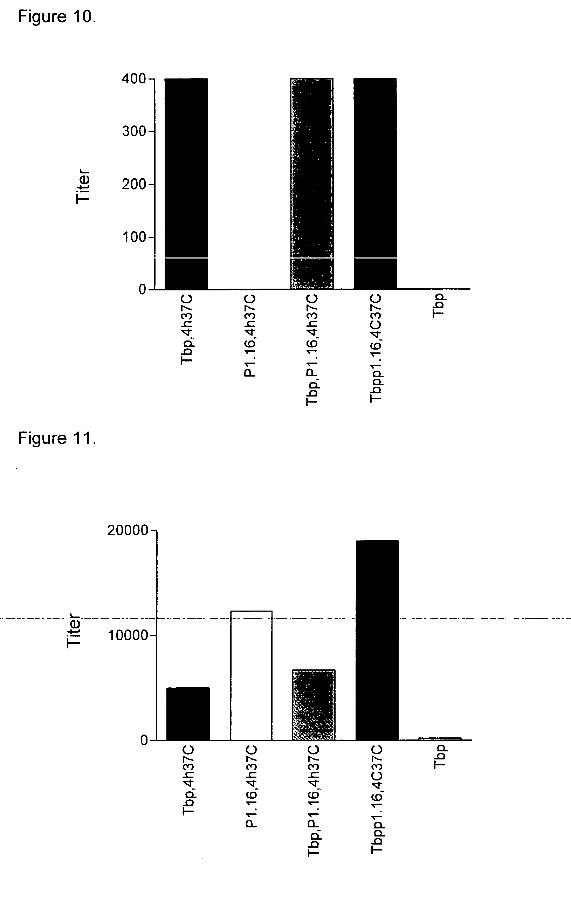

Figure 10. Transferrin binding inhibition by the presence of serum antibodies raised in the studied variants. The titer was calculated as the reciprocal value of the maximal dilution that promoted a 40% inhibition of total transferrin binding.
Figure 11. Graphical representation of titers by ELISA against P1.16 strain generated by immunization with different incorporation variants. The titer was calculated as the reciprocal value of the maximal dilution that duplicates the O.D of the pre-immune sera.

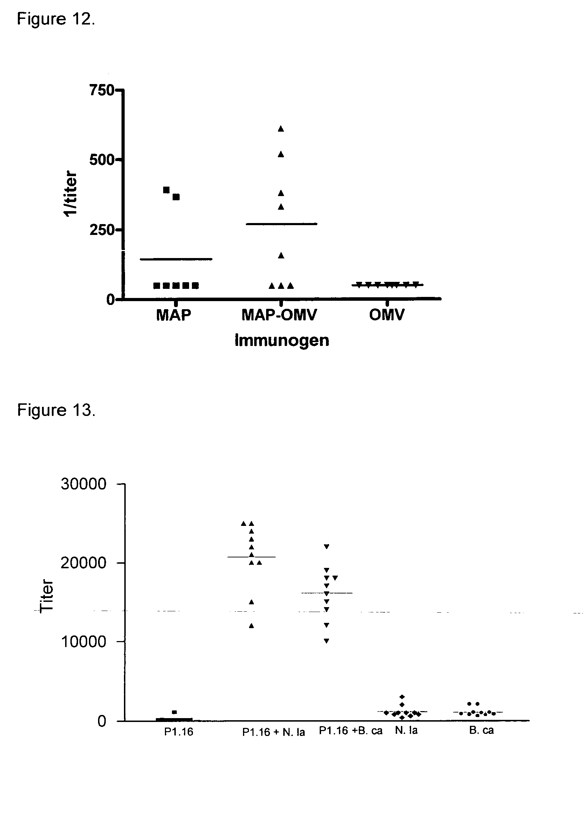

Figure 12. Anti-peptide VR2 antibody levels after immunization with a MAP of the VR2 of PorA. As coating agent a conjugate peptide-BSA was used.
Figure 13. Graphical representation of titers against OMVs of a P1.16 neisserial strain, detected in the sera raised by immunization with recombinant P1.16 alone, with the same protein incorporated into *N. lactamica* OMVs or *B. catarrhalis* OMV, or with OMVs alone. The titer was calculated as the reciprocal value of the maximal dilution that duplicates the O.D of the pre-immune sera.

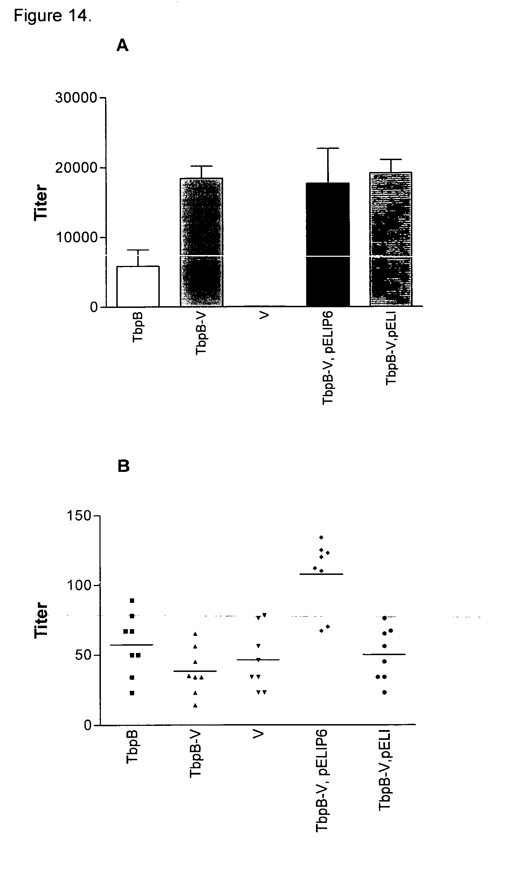

Figure 14. Graphical representation of titers measured against recombinant TbpB (A) and recombinant *Haemophilus influenzae* P6 (B), obtained after the evaluation of the sera raised with: TbpB, TbpB-OMV strain CU385 (TbpB-V), OMV strain CU385 (V), TbpB-OMV from strain CU385 co-formulated with pELIP6 (TbpB-V,pELlP6) and TbpB-OMV strain CU385 co-formulated with pELI (TbpB-V,pELI). The titer was calculated as the reciprocal value of the maximal dilution that duplicates the O.D of the pre-immune sera.

### Examples

The present invention is described herein through examples which in spite of being informative about the invention itself they do not represent, by any mean, a limit to the scope of the said invention.

### Example 1: Influence of the vesicle structure in OMV from Neisseria meningitidis, when administered by intranasal route, on its immunogenicity and protective capacity.

A comparative study between OMVs and NVOMPs (Non Vesicle forming outer membrane proteins) was carried out through the following experiment. Two groups of 10 mice each were immunized through the intranasal route (IN). Animals received 3 doses 7 days apart, with 50 ug of OMVs or NVOMPs, in a volume of 50 ul per animal. Detailed group composition of the immunogen is shown in Table 1.

**Table 1. Composition of the immunogen used for each group.**

| Group | OMVs | NVOMPs |
|---|---|---|
| 1 | 50µg | -- |
| 2 | -- | 50µg |

IgG levels against strain B385 OMVs after second and third doses were determined by ELISA. Results are presented in Table 2. Antibody titers were calculated as the reciprocal value of the maximal dilution that triplicates the O.D of the pre-immune sera. Statistical analysis was performed through a *t*-Student test.

**Table 2. ELISA and bactericidal titers after evaluation of the pooled sera.**

| | **Antibodies against OMVs strain CU385** | | **Functional activity** |
|---|---|---|---|
| Group | Titer (2^{nd} dose) | Titer (3^{rd} dose) | Bactericidal Titer |
| 1 | 3447 | 25600 | 1024 |
| 2 | 2395 | 5855 | 32 |

After the third dose a significantly higher titer was recorded in the group immunized with the OMVs, which demonstrates the influence of the vesicle structure in the induction of immune response by intranasal route. These results are also coherent with the results obtained in the bactericidal assay, and also presented in Table 2, as well as the results of the passive protection experiments in infant rats (Figure 1). The bactericidal titer was calculated as the reciprocal value of the maximal dilution that produces 50% killing of the total bacteria applied.

In both assays the functional activity of the immune response elicited against the outer membrane proteins is tested and in both cases the activity of the sera raised with the OMVs induce higher titers than the induced by immunization with NVOMPs.

### Example 2: Purification of recombinant PorA and insertion into N. meningitidis OMVs while maintaining intact the vesicle structure. Purification of recombinant protein PorA 7,16,9

By genetic engineering a clone expressing the recombinant PorA protein which contains the epitopes corresponding to subtypes 7, 16 and 9 in the same polypeptide, was obtained. The expressing clone was grown in LBA culture media at 37 °C for 8 hours in the presence of kanamicyn. After centrifugation, the cellular pellet was disrupted by sonication and the insoluble fraction was collected in order to be treated in the following way:
- Wash with Tris-EDTA, pH 8.0 buffer (TE), followed by a wash with TE + NaCl 0.1M, MgCl 0.8M, 0.5% NP40.
- Solubilization with a buffered carbonate-bicarbonate at pH 10.0 solution containing Urea 8M to a final protein concentration of 10 mg/ml.

After solubilization several steps were followed for chromatographic purification of the protein. Consequently the solubilized protein was diluted 1:2 with TE in order to achieve a 4M concentration of Urea and it was subsequently subjected to ionic exchange chromatography in Q-Sepharose. The purified protein is showed in Figure 2.

### Insertion of recombinant protein PorA 7,16,9 into OMV

In order to explore the conditions needed for protein insertion/re-folding into OMV six different variants were tested. In variant number II, incorporation of the protein was achieved by mixing it with the OMVs preparation in a 1:1 ratio in Tris-HCl 1M, 2mM EDTA, 1.2% sodium deoxycholate, and 20% de sucrose (solution B). After the mixing step, the sample was centrifuged. Variant number III was kept in the same conditions of Variant II but with an increased ratio protein:OMV of 2:1. In variant IV the mix was incubated 1.5 hours at room temperature (RT) prior centrifugation. In variants V and VI recombinant protein was folded by dilution into solution B, and only then mixed with the OMVs and centrifuged at 125 000g. Incorporation of recombinant protein was followed by electrophoresis of the assayed variants. The presence of a band with the same molecular weight of the recombinant protein in some variants was taken as an evidence of the incorporation. Further evidence was the recognition of the expected band by a monoclonal antibody (MAb) specific against P1.16 subtype, by western blotting, as it can be observed in Figure 3B.

In Table 3 it is shown the percent of incorporation calculated by ELISA using a sandwich capture ELISA assay with two MAbs, one anti-P1.9 subtype and a second one against the N-terminal region of the P64k protein, since this P64k epitope is fused to the first segment of the recombinant protein P1.7,16,9 and used as stabilizer for protein expression as well as immunological tag.

**Table No. 3 Percent of protein incorporation for each tested variant.**

| Variant N° | OMV | Protein_{rec} | Folding process | % of* incorporation |
|---|---|---|---|---|
| I | 5µg | - | - | - |
| II | 5µg | 5µg | mix, ultracentrifugation | 17 |
| III | 5µg | 10µg | mix, ultracentrifugation | 19.6 |
| IV | 5µg | 10µg | mix 1.30', | 18 |
| | | | ultracentrifugation | |
| V | 5µg | 10µg | Folding by dilution, mix, ultracentrifugation | 8 |
| VI | 5µg | 5µg | Folding by dilution, mix, ultracentrifugation | 14 |

After using different methods for measuring recombinant protein incorporation into OMVs, differences between the methods were recorded. However, differences were closely related to the method of quantification, being the quantitative ELISA the best of all, it was in agreement with the animal immunization experiments performed. All variants were analyzed by electron immuno-microscopy. Results of the electron microphotographs were in complete agreement with those obtained by the quantitative ELISA. In Figure 4 A, B and C results for variant IV are shown. As it can be seen the presence of the incorporated recombinant protein is demonstrated by the binding of the respective MAb labeled with gold particles. In all cases, it is obvious that the vesicle structure remained intact after the process of recombinant protein incorporation.

### Example 3: Evaluation of the immune response against recombinant PorA protein inserted into OMV from Neisseria meningitidis.

Once the variant of incorporation for recombinant P1.7, 16, 9 was selected, the insertion of the recombinant proteins P1.9, P1.16 and P1.7, 16 in OMVs preparations was conducted. These preparations were used as controls in an immunization schedule in order to evaluate the immune response generated against the protein P1.7, 16,9. These additional antigens were cloned and expressed in *E. coli* and were isolated from strains of *N. meningitidis* that express each of these subtypes given the variability that the PorA (also called P1) protein exhibits.

Recombinant proteins were refolded by dilution into Tris-HCl 1M, 2mM de EDTA, 1.2% sodium deoxycholate, and 20% sucrose, and incubated with OMVs 1.5 hours at RT before centrifugation. From this point forward, this method was chosen to insert protein antigens into the outer membrane vesicles of gram negative bacteria.

To verify the insertion of recombinant proteins into OMVs, Western-blot experiments, with MAbs specific to the subtypes employed, were carried out. In Figure 5 the result of the immunoidentification using MAb 1-33 specific for subtype P1.9 is shown and demonstrates the insertion of P1.9 and P1.7, 16, 9 into OMVs.

To evaluate the immunogenicity of the incorporated proteins, 40 Balb/C female mice (8-10 weeks old) were distributed into four groups of 10 animals each. Three subcutaneous immunizations, one week apart, were applied to these animals with blood extraction on the day of immunization and one week after the last dose. Samples were adjuvated with aluminum hydroxide to a final concentration of 40 µg per µg of protein.

**Table No. 4 Composition of immunogens used for each group.**

| Group | OMV of strain CU385 | Recombinant Protein |
|---|---|---|
| 1 | 5µg | 10µg (P1.9) |
| 2 | 5µg | 10µg (P1.16) |
| 3 | 5µg | 10µg (P1.7,16) |
| 4 | 5µg | 10µg (P17,16,9) |

After the third dose the existing specific IgG serum titer against the recombinant P1.7,16,9 protein was measured by ELISA. In the same way the recognition of OMVs by the respective sera against the were measured by using different *N. meningitidis* strains bearing the homologous P1 subtypes.
Statistical analysis was performed by the non-parametric Kruskal-Wallis test (with Dunn's multiple comparison post tests) due to the existence of non-homogeneous variance according to the Bartlett test.
Figure 6 represents the results obtained in the induction of serum antibodies against recombinant P1.7, 16,9. This assay was carried out by using pooled sera, and as it can be observed immunization with each of the inserted recombinant proteins was an effective mean to induce an immune response against the chimeric protein that posses all the P1 subtypes under study. The best results were generated when the same P1.7, 16,9 was used in the preparation.
When the response against natural antigens present in the homologous *N*. *meningitidis* strains was evaluated, it was observed a clear positive reaction against the homologous P1 subtype. An example is shown in Figure 7. Individual mouse sera from those animals immunized with the recombinant protein P1.7, 16,9 (inserted into OMVs) were able of recognizing the OMVs from strains expressing P1.7, P1.9, P1.16 and P1.7,16 subtypes.
To test the functional activity of the antisera against live *N. meningitidis* an experiment for testing the complement-mediated lysis, known as bactericidal assay, was performed. In Figure 8, the induced bactericidal titers against strains expresing P1.9, P1.16, P1.7, y P1.7, 16, respectively, are shown. Antibodies generated by immunization with P1.7, 16,9 re-folded in OMVs were bactericidal against all the heterologous strains bearing subtypes included in its original design.

### Example 4: Insertion of TbpB protein into OMV. Generation of a PorA-TbpB-OMV complex by co-refolding and evaluation of the immune response generated

The TbpB protein is a component of the human transferrin's receptor that is expressed by *N. meningitidis* and is an antigen of vaccine interest. Antigenic profile of TbpB proteins allowed their classification into two different groups or isotypes: I and II, which are also identifiable according to their different molecular weight.

The TbpB protein from strain B16B6 was cloned and expressed in *E. coli.* The recombinant protein was purified by chromatographic protocols described elsewhere. To refold this isotype I recombinant TbpB protein, the OMV from the Cuban strain B385 were chosen because it expresses a TbpB protein of isotype II and correspondingly is different from the B16B6 TbpB.
In order to refold the recombinant TbpB the protein was incubated 4 hours at 37 °C with either OMVs alone, recombinant P1.16 PorA plus OMVs, or a preformed mix of PorA and OMVs previously incubated 4 hours at 37°C. In all cases each preparation was subjected to centrifugation at 125 000 g.
To evaluate the immune response 50 female Balb/c mice (8-10 weeks old) were divided into 5 different groups (10 animals each) and subsequently immunized using the samples described in Table 5.

**Tabla No. 5 Composition of the immunogen for each group.**

| Group | OMV strain B385 (CU385) | Recombinant protein |
|---|---|---|
| 1 | 5µg | 10µg-TbpB, 4h at 37°C |
| 2 | 5µg | 10µg-P1.16, 4h at 37°C |
| 3 | 5µg | 10µg-(P1.16 y TbpB, 4h at 37°C) |
| 4 | 5µg | 10µg-(P1.16, 4h a 37°C + TbpB, 1 h at 4°C) |
| 5 | - | 10µg-TbpB |

Three immunizations were carried out by the SC route, with one week intervals, blood extractions the same day of inoculation and a final blood extraction one week after the last dose. Proteins were adjuvated with 40 µg aluminum hydroxide per µg of protein.
Elicited antibodies in the form of ELISA titers are presented in Figure 9. As coating antigens were used OMVs from strain B16B6 and recombinant protein.
The specificity of the raised anti-sera was demonstrated by ELISA for all the variants under analysis (Figure 9A). When TbpB protein is not exposed to 37 °C incubation the antibodies elicited are able to recognize the intact protein presented on its natural environment of B16B6 OMVs (Figure 9B).

Anti-TbpB antibodies, when functional, have a measurable blocking activity of transferrin binding on the human transferrin interaction with meningococcal outer membranes. Correspondingly, the sera elicited against the recombinant protein inserted into the OMVs of a heterologous strain were analyzed in a transferrin binding inhibition assay (Figure 10). The significant differences observed for the titers against the natural protein failed to correlate with the observed inhibition titers. All variants were able to induce blocking antibodies that were able to inhibit the binding of human transferrin to the meningococcal transferrin receptor present in the tested OMVs.
Additionally the combination of recombinant TbpB with recombinant PorA (subtype P1.16), both inserted into the OMV of an heterologous strain did not affect the immunogenicity of recombinant PorA in the PorA-TbpB-VME when they are incubated at 4°C (Figure 11).

### Example 5: Evaluation of the immune response induced against a synthetic peptide inserted into OMVs of Neisseria meningitidis.

In order to achieve higher immunogenicity, by mucosal route, a Multiple Antigen Peptide (MAP) containing the variable region 2 (VR2) (Garay, H.E *et al.* (2000) Disulfide bond polymerization of a cyclic peptide derived from the surface loop 4 of class 1 OMP of *Neisseria meningitidis. Lett. Pept. Sci.* 7:97-105) of the PorA porin of *N. meningitidis* present in strain CU385 (subtype 15) was incorporated into OMV of a heterologous *N. meningitidis* strain (233), with classification C: 2a:P1.5 (subtype 5). This antigenic combination was administered to mice *(n=8),* by the intranasal route, in a scheme of three doses, at two week intervals. Immunized groups are presented in Table 6.

**Table 6. Composition of immunogen per group.**

| Group | MAP | OMV |
|---|---|---|
| 1 | 50 µg | - |
| 2 | 50 µg | 10 µg |
| 3 | - | 10 µg |

Two weeks after the second and third doses, respectively, animals were sampled and sera were isolated. Specific titers against VR2 region of the porin under study were measured by ELISA, using VR2 peptide conjugated to bovine serum albumin. The results obtained, with mouse sera taken after two doses are shown in Figure 12. It can be appreciated that an statistical higher titer was elicited when the peptide was administered with the OMVs. Normally, those OMV are unable to induce a significant response against this peptide because they belong to an heterologous strain (group 3).
Additionally, immunobiot experiments were carried out in which 10µg per lane of B385 OMV proteins were eletrophoretically separated and subsequently transferred onto nitrocelulose membranes and incubated with pooled sera from each group. In the sera obtained two weeks after the last dose a greater reactivity against PorA protein (subtype 15) was scored by group 2, when compared with group 1, indicating that the reactivity against the parental protein was facilitated by the inclusion of the MAP into the OMVs of a strain of different subtype.

### Example 6: Evaluation of the immune response generated against recombinant PorA protein inserted in outer membrane vesicles of Neisseria lactamica and Bramhamella catarrhalis

OMVs were obtained from other gram-negative microorganisms, like *Neisseria lactamica* and *Bramhamella catarrhalis,* and they were used in the re-folding process of recombinant PorA protein (subtype 16) by following the procedures previously described.
In order to test the immunogenicity of the recombinant protein inserted in both OMVs, 50 female mice (8-10 weeks old) were divided into five groups (10 animals each), and were immunized according to Table 7. Three immunizing doses were applied by SC route, with two weeks intervals, and the animals were bleed on every immunization day and one week after the final third dose. The proteins were adjuvated with 40 µg of aluminum hydroxide per µg of protein.

**Table 7. Immunogen composition employed in each group.**

| Group | OMV *N. lactamica* | OMV *B. catarrhalis* | Recombinant P1.16 |
|---|---|---|---|
| 1 | | | 10µg |
| 2 | 5µg | | 10µg |
| 3 | | 5µg | 10µg |
| 4 | 5µg | | |
| 5 | | 5µg | |

After the third dose, serum antibody (IgG) titers against recombinant P1.16 protein were measured by ELISA, as well as antibodies against the parental protein present in OMVs. Statistical analyses of the results were generated by using one way ANOVA. Comparison among treatment's mean was performed through the Newman-Keuls Multiple Comparison Test.
Figure 13 graphically shows the results obtained after the immunization regarding antibody levels against natural protein P1.16 present in the OMVs of the given strain. Recombinant protein adjuvated in aluminum hydroxide failed to elicit antibodies with a detectable recognition of native subtype 16 porin. The highest titer against this native protein was induced after the immunization with antigen inserted in *N. lactamica* OMV, with antigen inserted in *B. catarrhalis* OMV coming in second level. There were also significant differences among the titers induced after immunization with the recombinant protein inserted into OMV and those elicited after immunization with OMVs, coming from these two microorganisms, alone.

### Example 7: Evaluation of the immune response induced against a formulation composed by recombinant TbpB, inserted into B385 OMVs, and a DNA immunization vector.

A plasmid codifying for the P6 protein from *Haemophilus influenzae,* was constructed, correspondingly labeled as pELIP6, and subsequently employed in DNA immunization experiments. It was purified the necessary material for the inclusion of this plasmid into a formulation containing recombinant TbpB (B16B6) inserted into OMVs of *N. meningitidis* strain CU385. As a control, the pELI vector was obtained, which is similar to pELlP6 but lacking the P6 codifying segment.

For the immunogenicity testing of the formulations 40 female mice (8-10 weeks old) were distributed into five groups (of 8 animals each) accordingly to Table 8. Four immunizations were carried out subcutaneously, with three week intervals, and blood extrations were made on day 0 and three weeks after the fourth inoculation. Proteins were adjuvated with 40 µg of aluminum hydroxide per µg of protein.

**Table 8. Immunogen composition by group.**

| Group | OMV CU385 | Recombinant TbpB | pELIP6 | pELI |
|---|---|---|---|---|
| 1 | | 10 µg | | |
| 2 | 5µg | 10 µg | | |
| 3 | 5µg | | | |
| 4 | 5µg | 10 µg | 50 µg | |
| 5 | 5µg | 10 µg | | 50 µg |

The sera extracted at the end of the immunization schedule were evaluated and their titers against recombinant TbpB were recorded. Furthermore, the antibody titers against the P6 protein were also measured. The P6 recombinant antigen was previously purified from a genetically modified *E. coli* strain.
The statistical analisis was performed by one way ANOVA. Comparison among treatment's mean was performed through a Newman Keuls Multiple Comparison Test.
Figure 14 shows the immunization results regarding the antibody levels against recombinant TbpB protein and recombinant P6 protein, respectively. Higher titers against recombinant TbpB were elicited when the protein was inserted into OMVs of a heterologous strain. The presence of pELIP6 plasmid in the formulation did not affect the immunogenicity of the recombinant TbpB protein.
Similarly, the plasmid codifying for P6 induced an antibody response according to the expected levels after its use as immunogen in the presence of the complex TbpB-OMV. This response was obviously higher than the one induced by the group containing a similar formulation but with the control, the pELI plasmid vector.

## Claims

1. Method for antigen incorporation into bacterial outer membrane vesicles **characterized by** the formation of a non-covalent complex between these antigens and outer membrane proteins form gram-negative bacteria, being such complex generated by co-folding while maintaining intact the vesicle structure.

2. A method according to claim 1 wherein said outer membrane preparation is obtained from gram-negative bacteria belonging to the *Neisseriaceae* family or to the *Bramhamella catarrhalis* species.

3. A method according to claim 2 wherein said outer membrane preparation is obtained from *Neisseria meningitidis* and *Neisseria lactamica.*

4. A method according to claims 1-3 wherein said protein antigen is of natural, recombinant or synthetic origin.

5. A vaccine composition obtained according to claim 1, for its administration by parenteral or mucosal routes, **characterized** because comprises a complex formed by a protein antigen and a preparation of outer membrane proteins of gram-negative bacteria, being such complex generated by co-folding while maintaining intact the vesicle structure in combination with pharmaceutically acceptable excipient.

6. A vaccine composition obtained according to claim 5 wherein said outer membrane preparation is obtained from gram-negatives bacteria belonging to the *Neisseriaceae* family or to the *Bramhamella catarrhalis* species.

7. A vaccine composition obtained according to claim 6 wherein said outer membrane preparation is obtained from *Neisseria meningitidis* and *Neisseria lactamica.*

8. A vaccine composition obtained according to claims 5-7 wherein said protein antigen is of natural, recombinant or synthetic origin.

9. A vaccine composition obtained according to claims 5-7 **characterized** for including also capsular bacterial polysaccharides.

10. A vaccine composition obtained according to claims 5-7 **characterized** for including also conjugated capsular bacterial polysaccharides.

11. A vaccine composition obtained according to claims 5-7 **characterized** for including also a nucleic acid as antigen

12. A vaccine composition obtained according to claims 5-11 for its therapeutic, or prophylactic use in humans.
